# EUROPEAN PATENT SPECIFICATION

(11) **EP 3 673 083 B1**
(45) Date of publication and mention of the grant of the patent: **13.07.2022**
(21) Application number: 18765575.8
(22) Date of filing: 24.08.2018
(51) Int. Cl.: G01N 35/00, G16H 10/40, G16H 40/63, B01L 3/00, C12Q 1/6825, G01N 27/327, G01N 33/543

(54) **ANALYSIS SYSTEM WITH CARTRIDGE AND METHOD FOR TESTING A SAMPLE**
ANALYSESYSTEM MIT KARTUSCHE UND VERFAHREN ZUM TESTEN EINER PROBE
SYSTÈME D'ANALYSE AVEC CARTOUCHE ET MÉTHODE DE TEST D'UN ÉCHANTILLON

(30) Priority: 05.10.2017 EP 17020454
(43) Date of publication of application: 01.07.2020
(73) Proprietor: Boehringer Ingelheim Vetmedica GmbH, 55216 Ingelheim am Rhein (DE)
(72) Inventor: SCHOEDER, Heinz, 55216 Ingelheim am Rhein (DE); SCHMOLKE, Hannah, 55216 Ingelheim am Rhein (DE); NIEMEYER, Axel, 55216 Ingelheim am Rhein (DE)
(74) Representative: Von Rohr Patentanwälte Partnerschaft mbB
(86) International application number: PCT/EP2018/072872
(87) International publication number: WO 2019/068392

(56) References cited:
- WO-A1-2016/122705
- WO-A1-2018/065113
- WO-A1-2018/065114
- WO-A1-2018/067872
- WO-A1-2018/067878
- US-A- 5 004 583
- US-A1- 2014 273 187
- US-A1- 2014 296 089
- US-A1- 2016 047 832

## Description

The present invention relates to an analysis system according to the preamble of claim 1 and to a method according to the preamble of claim 11.

Preferably, the present invention deals with analysing and testing a preferably biological sample, in particular from a human or animal, particularly preferably for analytics and diagnostics, for example with regard to the presence of diseases and/or pathogens and/or for determining blood counts, antibodies, hormones, steroids or the like. Therefore, the present invention is in particular within the field of bioanalytics. A food sample, environmental sample or another sample may optionally also be tested, in particular for environmental analytics or food safety and/or for detecting other substances.

Preferably, by means of the cartridge, at least one analyte (target analyte) of a sample can be determined, identified or detected. In particular, the sample can be tested for qualitatively or quantitatively determining at least one analyte, for example in order for it to be possible to detect or identify a disease and/or pathogen.

Within the meaning of the present invention, analytes are in particular nucleic-acid sequences, in particular DNA sequences and/or RNA sequences, or proteins, in particular antigens and/or antibodies. In particular, by means of the present invention, nucleic-acid sequences or proteins can be determined, identified or detected as the analytes of a sample. More particularly preferably, the present invention deals with systems, devices and other apparatuses for carrying out a nucleic-acid assay for detecting or identifying a nucleic-acid sequence or a protein assay for detecting or identifying a protein.

The present invention deals in particular with what are known as point-of-care systems, i.e. in particular with mobile systems, devices and other apparatuses, and deals with methods for carrying out tests on a sample at the sampling site and/or independently or away from a central laboratory or the like. Preferably, point-of-care systems can be operated autonomously and/or independently of a mains network for supplying electrical power.

US 5,096,669 discloses a point-of-care system for testing a biological sample, in particular a blood sample. The system comprises a single-use cartridge and an analysis device. Once the sample has been received, the cartridge is inserted into the analysis device in order to carry out the test. The cartridge comprises a microfluidic system and a sensor apparatus comprising electrodes, which apparatus is calibrated by means of a calibration liquid and is then used to test the sample.

Furthermore, WO 2006/125767 A1 discloses a point-of-care system for integrated and automated DNA or protein analysis, comprising a single-use cartridge and an analysis device for fully automatically processing and evaluating molecular-diagnostic analyses using the single-use cartridge. The cartridge is designed to receive a sample, in particular blood, and in particular allows cell disruption, PCR and detection of PCR amplification products, which are bonded to capture molecules and provided with a label enzyme, in order for it to be possible to detect bonded PCR amplification products or nucleic-acid sequences as target analytes in what is known as a redoxcycling process.

US2017/0296089 relates to systems and methods for multi-analysis.

In point-of-care systems, it is important that the analysis systems and cartridges used are constructed in a simple and robust manner and allow multiple measurements.

The problem addressed by the present invention is to provide a cartridge, an analysis system and a method for testing a sample, wherein a simple, robust and/or cost-effective construction and/or better testing are made possible or facilitated.

The above problem is solved by an analysis system and a method as claimed in the appended claims 1 and 11.

Advantageous developments are the subject of the dependent claims.

Preferably, the cartridge or analysis system or an analysis device thereof comprise a sensor apparatus for electrochemically detecting or identifying one or more analytes of the sample and/or for detecting or identifying one or more analytes of the sample by specifically bonding the analytes(s) to one or more capture molecules as primary measurement. In particular, the capture molecules allow a specific bonding of the respective analytes or amplification products thereof.

The sensor apparatus or a sensor array thereof preferably comprises multiple sensor fields and/or electrodes for specifically bonding and/or detecting the one or more analytes to be detected or measured. Further, the sensor apparatus preferably is configured for electrical or electrochemical detection of analytes of the sample, in particular by redoxcycling.

According to one aspect of the present disclosure, but not part of the invention, the cartridge comprises preferably a measurement chamber for detecting or identifying further analytes, compounds, material characteristics of the sample by optical measurement and/or without specific bonding, i. e. for additional measurement. This allows a very simple, robust and/or cost-effective construction as well as simple handling of the sample. Further, the proposed solution allows multiple measurements and/or different kinds of measurements to be combined, in particular in a point-of-care system.

Preferably, the measurement chamber is separate from the sensor apparatus and/or sensor compartment. This allows optimization for the different measurements, i. e. for the primary measurement on one hand and the additional measurement on the other hand.

Alternatively, the measurement chamber for the additional measurement may be integrated into or formed by or within the sensor apparatus or sensor compartment. This allows in particular a very compact and/or simple construction.

Preferably, the cartridge or sensor apparatus is designed to perform a nucleic-acid sequence assay and/or a protein assay, in particular by means of the sensor apparatus.

The measurement chamber allows at least one or more different or additional measurements without specific bonding and/or without electrochemical detection and/or by optical measurement or spectroscopy.

According to another aspect, which can be realised independently, the analysis system is adapted to detect or identify one or more analytes of the sample by electrochemical measurement, in particular redoxcycling, and/or by specifically bonding the analytes to one ore more capture molecules (also called primary measurement), wherein the analysis system is adapted for detecting or identifying further analytes, compounds or material characteristics of the sample by optical measurement and/or without specific bonding, i. e. the system allows an additional measurement. This allows a very simple, robust and/or cost-effective construction as well as simple handling of the sample. Further, the proposed solution allows multiple measurements and/or different kinds of measurements to be combined, in particular in a point-of-care system.

Preferably, the additional measurement for detecting or identifying further analytes, compounds and/or material characteristics is conducted or performed in a measurement chamber, in particular separately from the sensor apparatus on the cartridge. The measurement chamber can be formed by the cartridge or a separate sample carrier.

Preferably, the proposed analysis system comprises an analysis device for connecting or receiving the cartridge with the sample.

Preferably, the analysis device comprises a measurement apparatus, such as a (optical) spectrometer, for detecting or identifying the further analytes, compounds or material characteristics of the sample, i. e. for the additional measurement.

If the measurement chamber for the additional measurement is formed separately from the cartridge, in particular in or by a (separate) sample carrier, the analysis system or device comprises preferably an interface for connecting the sample carrier in order to perform the additional measurement for detecting or identifying the further analytes, compounds and/or material characteristics of the sample, in particular by optical measurement or without specific bonding.

A method according to the present invention for testing an in particular biological sample is characterized in that the primary measurement and the additional measurement are conducted and/or in that the analysis device detects or identifies further analytes, compounds and/or material characteristics of the sample by optical measurement and/or without specific bonding.

The additional measurement provides further information about the sample in addition to the primary measurements for detecting or identifying one or more analytes electrochemically and/or by specific bonding to one or more capture molecules, in particular performed in the cartridge and/or by the sensor apparatus on the cartridge. Thus, a better characterization or testing of the sample is enabled or facilitated.

In particular, the (additional) measurement can be configured for detecting or measuring other or further analytes, compounds, material characteristics or the like and/or can include impedance measurement, capacitance measurement, optical spectrometric measurement, mass spectrometric measurement, and/or tomography like MRT. In this regard, the measurement apparatus or analysis device can comprise or form an arrangement enabling such measurement(s).

An optical measurement or the sensor apparatus, such as a spectrometer, can be realized independently of the cartridge and/or can form part of the analysis device.

Preferably, the sensor apparatus comprises a sensor compartment and the amplification products and/or different groups are bonded in the (same) sensor compartment to the corresponding capture molecules in particular at different hybridisation temperatures. This allows in particular a very compact and simple realization and/or detection or identification of a multiplicity of analytes, amplification products and/or groups by means of or within one sensor apparatus or sensor compartment.

The analytes and/or amplification products may be bonded at different hybridisation temperatures and/or are preferably detected in a single or common detection process.

Particularly preferably, the sensor apparatus is only used a single time for a process for detecting said analytes and/or amplification products, electrochemical determination preferably taking place in particular simultaneously for all the bonded amplification products. This allows very rapid and efficient testing.

It is proposed that different analytes and/or amplification products of different analytes can be very efficiently bonded in succession by hybridisation temperatures, to preferably immobilised capture molecules, particularly preferably on or in a sensor apparatus, in order for it to be possible to measure and/or determine or detect a particularly large number of different amplification products at the same time, in particular in a single or common detection process.

In the context of the present invention, it is thus possible to test analytes and/or amplification products, that are produced and/or amplified in parallel and have different hybridisation temperatures, in a single detection process and at the same time with high specificity.

Preferably, different analytes and/or amplification products or groups thereof are initially, in particular simultaneously and/or in parallel, produced by means of an amplification reaction, in particular PCR, in preferably different PCR chambers and/or reaction cavities, and are then bonded to the capture molecules in succession at different hybridisation temperatures.

In particular, it is provided that the first, second and optional third group are produced in different reaction cavities. It may however also be provided that the analytes are amplified by means of an amplification reaction, in particular PCR, in a common PCR chamber and/or reaction cavity, and/or that the amplification products are produced in a common reaction cavity, and the subsequent hybridisation to the capture molecules is carried out at different, in particular decreasing, hybridisation temperatures.

Preferably, a plurality of amplification reactions, in particular PCRs, run simultaneously, in parallel or independently from one another during the test.

Preferably, different amplification reactions, in particular PCRs with different primers, are provided or carried out.

Within the meaning of the present invention, amplification reactions are in particular molecular-biological reactions in which an analyte is amplified/copied and/or in which amplification products, in particular nucleic-acid products, of an analyte are produced. Particularly preferably, PCRs are amplification reactions within the meaning of the present invention.

"PCR" stands for polymerase chain reaction and is a molecular-biological method by means of which certain analytes, in particular portions of RNA or DNA, of a sample are amplified, preferably in several cycles, using polymerases or enzymes, in particular in order to then test and/or detect the amplification products or nucleic-acid products. If RNA is intended to be tested and/or amplified, before the PCR is carried out, a cDNA is produced starting from the RNA, in particular using reverse transcriptase. The cDNA is used as a template for the subsequent PCR.

Preferably, during a PCR, a sample is first denatured by the addition of heat in order to separate the strands of DNA or cDNA. Preferably, primers or nucleotides are then deposited on the separated single strands of DNA or cDNA, and a desired DNA or cDNA sequence is replicated by means of polymerase and/or the missing strand is replaced by means of polymerase. This process is preferably repeated in a plurality of cycles until the desired quantity of the DNA or cDNA sequence is available.

For the PCR, marker primers are preferably used, i.e. primers which (additionally) produce a marker or a label, in particular biotin, on the amplified analyte. This allows or facilitates detection. Preferably, the primers used are biotinylated and/or comprise or form in particular covalently bonded biotin as the label.

The proposed analysis system or its cartridge for testing an in particular biological sample preferably comprises the sensor apparatus for detecting nucleic-acid sequences and/or in particular amplified analytes and/or amplification products, the sensor apparatus preferably comprising immobilised capture molecules for bonding the sequences, analytes and/or amplification products.

Preferably, the sensor apparatus comprises a sensor compartment, wherein the capture molecules are arranged or immobilized in the (same) sensor compartment of the sensor apparatus so that the different analytes, amplification products and/or groups can be bonded within the (same) sensor apparatus or sensor compartment at different hybridisation temperatures. This allows in particular a very compact and simple realization and/or detection or identification of a multiplicity of analytes, amplification products and/or groups by means of or within one sensor apparatus or sensor compartment.

The capture molecules are in particular oligonucleotide probes, which are preferably immobilised on the sensor, sensor array and/or electrodes preferably by a spacer, in particular a C6 spacer. The formation of structures that disrupt hybridisation, e.g. hairpin structures, can be prevented by the preferred bonding of the capture molecules by spacers.

Within the meaning of the present invention, the term "detector molecules" is preferably understood to mean molecules that bond specifically to the marker or label of the primers used to amplify the analytes and/or analytes or amplification products provided therewith, and thus allow the detection thereof.

In particular, the detector molecules may be enzyme conjugates and/or immunoconjugates, which bond specifically to the marker or label, in particular biotin, and comprise a reporter enzyme for converting a substrate.

In the context of the present invention, the detector molecules are preferably based on streptavidin, which has a high affinity for biotin, and/or alkaline phosphatase, which can convert non-reactive phosphate monoesters to electrochemically active molecules and phosphate.

Preferably, a detection system is used, where the label is based on biotin and where the detector molecules are based on streptavidin/alkaline phosphatase. However, other detector molecules can also be used.

The analysis system is in particular portable, mobile and/or is a point-of-care system and/or can be used in particular at the sampling site and/or away from a central laboratory.

The analysis system preferably comprises an analysis device and/or at least one cartridge for testing the sample.

The term "analysis device" is preferably understood to mean an instrument which is in particular mobile and/or can be used on site, and/or which is designed to chemically, biologically and/or physically test and/or analyse a sample or a component thereof, preferably in and/or by means of a cartridge. In particular, the analysis device controls the testing of the sample in the cartridge.

Particularly preferably, the analysis device is designed to receive the cartridge or to connect said cartridge.

The term "cartridge" is preferably understood to mean a structural apparatus or unit designed to receive, to store, to physically, chemically and/or biologically treat and/or prepare and/or to measure a sample, preferably in order to make it possible to detect, identify or determine at least one analyte of the sample.

A cartridge within the meaning of the present invention preferably comprises a fluid system having a plurality of channels, cavities and/or valves for controlling the flow through the channels and/or cavities.

In particular, within the meaning of the present invention, a cartridge is designed to be at least substantially planar and/or card-like, in particular is designed as a (micro)fluidic card and/or is designed as a main body or container that can preferably be closed and/or said cartridge can be inserted and/or plugged into a proposed analysis device when it contains the sample.

The above-mentioned aspects and features of the present disclosure 2. and the aspects and features of the present disclosure that will become apparent from the claims and the following description can in principle be implemented independently from one another, but also in any combination or order.

Other aspects, advantages, features and properties of the present disclosure will become apparent from the claims and the following description of embodiments with reference to the drawings, in which:
- Fig. 1: is a schematic section through a proposed analysis system or analysis device comprising a proposed cartridge received therein;
- Fig. 2: is a schematic view of the cartridge according to another embodiment;
- Fig. 3: is a schematic front view of a proposed sensor apparatus of the analysis system and/or cartridge;
- Fig. 4: is an enlarged detail from Fig. 3 illustrating a sensor field of the sensor apparatus;
- Fig. 5: is a schematic rear view of the sensor apparatus;
- Fig. 6: is a schematic sectional view of the sensor apparatus;
- Fig. 7: is a partial, schematic enlargement of Fig. 1; and
- Fig. 8: is a schematic section of the analysis system or device according to another embodiment with an additional sample carrier.

In the Figures, which are only schematic and sometimes not to scale, the same reference signs are used for the same or similar parts and components, corresponding or comparable properties and advantages being achieved even if these are not repeatedly described.

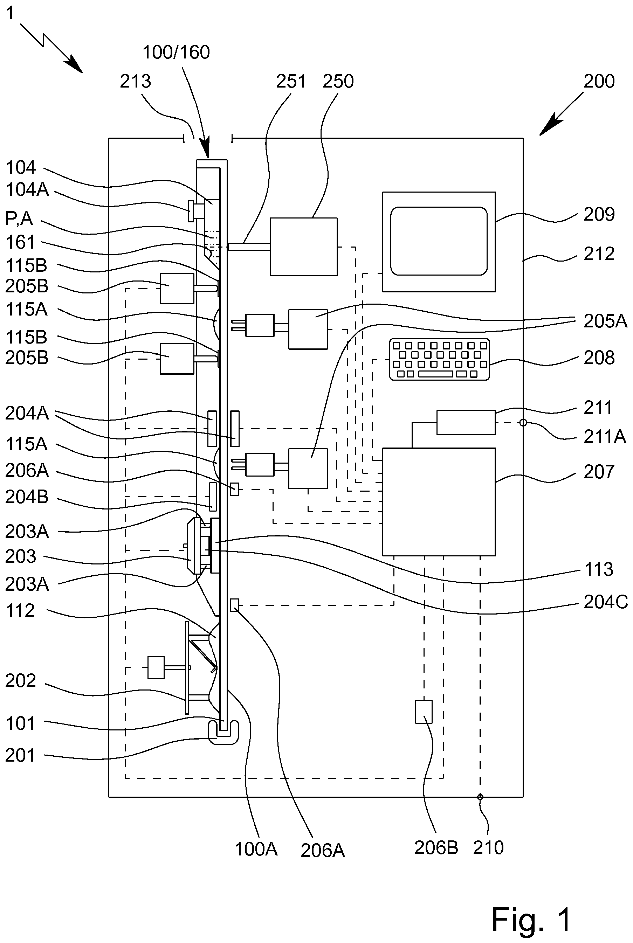

Fig. 1 is a highly schematic view of a proposed analysis system 1 and analysis device 200 for testing an in particular biological sample P, preferably by means of or in an apparatus or cartridge 100.

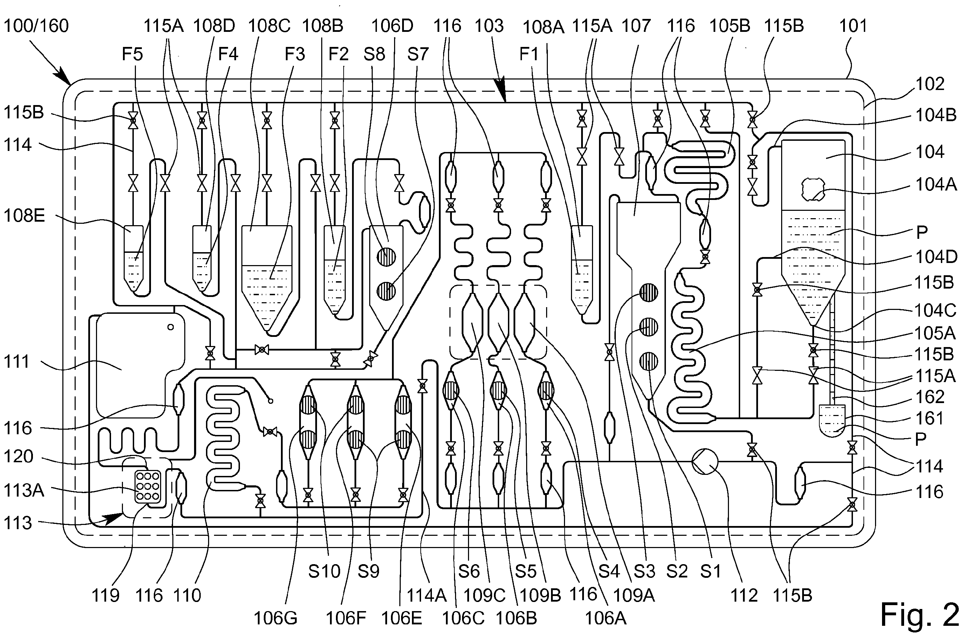

Fig. 2 is a schematic view of a preferred embodiment of the proposed apparatus or cartridge 100 for testing the sample P. The apparatus or cartridge 100 in particular forms a handheld unit, and in the following is merely referred to as a cartridge.

The term "sample" is preferably understood to mean the sample material to be tested, which is in particular taken from a human or animal. In particular, within the meaning of the present invention, a sample is a fluid, such as saliva, blood, urine or another liquid, preferably from a human or animal, or a component thereof. Within the meaning of the present invention, a sample may be pretreated or prepared if necessary, or may come directly from a human or animal or the like, for example. A food sample, environmental sample or another sample may optionally also be tested, in particular for environmental analytics, food safety and/or for detecting other substances, preferably natural substances, but also biological or chemical warfare agents, poisons or the like.

Preferably, the analysis system 1 or analysis device 200 controls the testing of the sample P in particular in or on the cartridge 100 and/or is used to evaluate the testing or the collection, processing and/or storage of measured values from the test.

By means of the proposed analysis system 1 or analysis device 200 or by means of the cartridge 100 and/or using the proposed method for testing the sample P, preferably an analyte A of the sample P, in particular a nucleic-acid product, such as a certain nucleic-acid sequence, or particularly preferably a plurality of analytes A of the sample P, can be determined, identified or detected. Said analytes are in particular detected and/or measured not only qualitatively, but particularly preferably also quantitatively.

Therefore, the sample P can in particular be tested for qualitatively or quantitatively determining at least one analyte A, for example in order for it to be possible to detect a disease and/or pathogen or to determine other values, which are important for diagnostics, for example.

Particularly preferably, a molecular-biological test is made possible by means of the analysis system 1 and/or analysis device 200 and/or by means of the cartridge 100.

Particularly preferably, a molecular and/or PCR assay, in particular for detecting DNA and/or RNA, i.e. nucleic-acid products and/or sequences, is made possible and/or carried out.

Preferably, the sample P or individual components of the sample P or analytes A can be amplified if necessary, in particular by means of PCR, and tested, identified or detected in the analysis system 1, analysis device 200 and/or in the cartridge 100. Preferably, amplification products V of the analyte A or analytes A are thus produced.

The analytes A and/or amplification products V of the sample P, in particular the nucleic-acid products, which are amplified in particular by means of PCR, in particular have a length of at least 20 or 50, particularly preferably 80 or 100, and/or at most 300 or 280, particularly preferably 250 or 220, nucleotides. However, it may also be provided that shorter or longer amplification products V are produced in particular by means of PCR.

In the following, further details are first given on a preferred construction of the cartridge 100, with features of the cartridge 100 preferably also directly representing features of the analysis system 1, in particular even without any further explicit explanation.

The cartridge 100 is preferably at least substantially planar, flat and/or plate-shaped and/or card-like.

The cartridge 100 preferably comprises an in particular at least substantially flat, planar, plate-shaped and/or card-like main body 101, the main body 101 in particular being made of and/or injection-moulded from plastics material, particularly preferably polypropylene.

The cartridge 100 preferably comprises at least one film or cover 102 for covering the main body 101 and/or cavities and/or channels formed therein at least in part, in particular on the front 100A, and/or for forming valves or the like, as shown by dashed lines in Fig. 2.

The analysis system 1 or cartridge 100 or the main body 101 thereof, in particular together with the cover 102, preferably forms and/or comprises a fluidic system 103, referred to in the following as the fluid system 103.

The cartridge 100 and/or the fluid system 103 thereof is preferably at least substantially vertically oriented in the operating position and/or during the test, in particular in the analysis device 200, as shown schematically in Fig. 1. In particular, the main plane or surface extension of the cartridge 100 thus extends at least substantially vertically in the operating position.

The cartridge 100 and/or the fluid system 103 preferably comprises a plurality of cavities, in particular at least one receiving cavity 104, at least one metering cavity 105, at least one intermediate cavity 106, at least one mixing cavity 107, at least one storage cavity 108, at least one reaction cavity 109, at least one intermediate temperature-control cavity 110 and/or at least one collection cavity 111, as shown in Fig. 1.

The cartridge 100 and/or the fluid system 103 also preferably comprises at least one pump apparatus 112 and/or at least one sensor apparatus 113.

Some, most or all of the cavities are preferably formed by chambers and/or channels or other depressions in the cartridge 100 and/or the main body 101, and particularly preferably are covered or closed by the film or cover 102. However, other structural solutions are also possible.

In the example shown, the cartridge 100 or the fluid system 103 preferably comprises two metering cavities 105A and 105B, a plurality of intermediate cavities 106A to 106G, a plurality of storage cavities 108A to 108E and/or a plurality of reaction cavities 109, which can preferably be loaded independently from one another, in particular a first reaction cavity 109A, a second reaction cavity 109B and an optional third reaction cavity 109C, as can be seen in Fig. 2.

The reaction cavity/cavities 109 is/are used in particular to carry out an amplification reaction, in particular PCR, or several, preferably different, amplification reactions, in particular PCRs. It is preferable to carry out several, preferably different, PCRs, i.e. PCRs having different primer combinations or primer pairs, in parallel and/or independently and/or in different reaction cavities 109.

The amplification products V and/or other portions of the sample P forming in the one or more reaction cavities 109 can be conducted or fed to the connected sensor apparatus 113, in particular by means of the pump apparatus 112.

The sensor apparatus 113 is used in particular for detecting, particularly preferably qualitatively and/or quantitatively determining, the analyte A or analytes A of the sample P, in this case particularly preferably the amplification products V of the analytes A. Alternatively or additionally, however, other values may also be collected or determined.

In particular, the pump apparatus 112 comprises or forms a tube-like or bead-like raised portion, in particular by means of the film or cover 102, particularly preferably on the back of the cartridge, as shown schematically in Fig. 1.

The cartridge 100, the main body 101 and/or the fluid system 103 preferably comprise a plurality of channels 114 and/or valves 115, as shown in Fig. 2.

By means of the channels 114 and/or valves 115, the cavities 104 to 111, the pump apparatus 112 and/or the sensor apparatus 113 can be temporarily and/or permanently connected and/or separated from one another, as required and/or optionally or selectively, in particular such that they are controlled by the analysis system 1 or the analysis device 200.

The cavities 104 to 111 are preferably each fluidically linked by a plurality of channels 114. Particularly preferably, each cavity is linked or connected by at least two associated channels 114, in order to make it possible for fluid to fill, flow through and/or drain from the respective cavities as required.

The fluid transport or the fluid system 103 is preferably not based on capillary forces, or is not exclusively based on said forces, but in particular is essentially based on the effects of gravity and/or pumping forces and/or compressive forces and/or suction forces that arise, which are particularly preferably generated by the pump or pump apparatus 112. In this case, the flows of fluid or the fluid transport and the metering are controlled by accordingly opening and closing the valves 115 and/or by accordingly operating the pump or pump apparatus 112, in particular by means of a pump drive 202 of the analysis device 200.

Preferably, each of the cavities 104 to 110 has an inlet at the top and an outlet at the bottom in the operating position. Therefore, if required, only liquid from the respective cavities can be removed via the outlet.

In particular, the liquid-containing cavities, particularly preferably the storage cavity/cavities 108, the mixing cavity 107 and/or the receiving cavity 104, are each dimensioned such that, when said cavities are filled with liquid, bubbles of gas or air that may potentially form rise upwards in the operating position, such that the liquid collects above the outlet without bubbles. However, other solutions are also possible here.

Preferably, at least one valve 115 is assigned to each cavity, the pump apparatus 112 and/or the sensor apparatus 113 and/or is arranged upstream of the respective inlets and/or downstream of the respective outlets.

Preferably, the cavities 104 to 111 or sequences of cavities 104 to 111, through which fluid flows in series or in succession for example, can be selectively released and/or fluid can selectively flow therethrough by the assigned valves 115 being actuated, and/or said cavities can be fluidically connected to the fluid system 103 and/or to other cavities.

In particular, the valves 115 are formed by the main body 101 and the film or cover 102 and/or are formed in another manner, for example by additional layers, depressions or the like.

Particularly preferably, one or more valves 115A are provided which are preferably tightly closed initially or when in storage, particularly preferably in order to seal liquids or liquid reagents F, located in the storage cavities 108, and/or the fluid system 103 from the open receiving cavity 104 in a storage-stable manner.

Preferably, an initially closed valve 115A is arranged upstream and downstream of each storage cavity 108. Said valves are preferably only opened, in particular automatically, when the cartridge 100 is actually being used and/or while inserting the cartridge 100 into the analysis device 200.

A plurality of valves 115A, in particular three valves in this case, are preferably assigned to the receiving cavity 104 when an optional intermediate connection 104D is provided in addition to an inlet 104B and an outlet 104C, for example in order for it to be possible to optionally discharge or remove a supernatant of the sample P, such as blood serum or the like. Depending on the use, in addition to the valve 115A on the inlet 104B, then preferably only the valve 115A either at the outlet 104C or at the intermediate connection 104D is opened.

The valves 115A assigned to the receiving cavity 104 seal the fluid system 103 and/or the cartridge 100 in particular fluidically and/or in a gas-tight manner until the sample P is inserted and the receiving cavity 104 or a connection 104A of the receiving cavity 104 is closed.

As an alternative or in addition to the valves 115A (which are initially closed), one or more valves 115B are preferably provided which are not closed in a storage-stable manner and/or which are open initially and/or which can be closed by actuation. These valves are used in particular to control the flows of fluid during the test.

The cartridge 100 is preferably designed as a microfluidic card and/or the fluid system 103 is preferably designed as a microfluidic system. In the present invention, the term "microfluidic" is preferably understood to mean that the respective volumes of individual cavities, some of the cavities or all of the cavities 104 to 111 and/or channels 114 are, separately or cumulatively, less than 5 ml or 2 ml, particularly preferably less than 1 ml or 800 µl, in particular less than 600 µl or 300 µl, more particularly preferably less than 200 µl or 100 µl.

Particularly preferably, a sample P having a maximum volume of 5 ml, 2 ml or 1 ml can be introduced into the cartridge 100 and/or the fluid system 103, in particular the receiving cavity 104.

Reagents and liquids which are preferably introduced or provided before the test in liquid form as liquids or liquid reagents F and/or in dry form as dry reagents S are required for testing the sample P, as shown in the schematic view according to Fig. 2.

Furthermore, other liquids F, in particular in the form of a wash buffer, solvent for dry reagents S or a substrate SU, for example in order to form detection molecules and/or a redox system, are also preferably required for the test, the detection process and/or for other purposes and are in particular provided in the cartridge 100, i.e. are likewise introduced before use, in particular before delivery. At some points in the following, a distinction is not made between liquid reagents and other liquids, and therefore the respective explanations are accordingly also mutually applicable.

The analysis system 1 or the cartridge 100 preferably contains all the reagents and liquids required for carrying out one or more amplification reactions or PCRs and/or for carrying out the test, and therefore, particularly preferably, it is only necessary to receive the optionally pretreated sample P.

The cartridge 100 and/or the fluid system 103 preferably comprises a bypass 114A that can optionally be used, in order for it to be possible, if necessary, to conduct or convey the sample P or components thereof past the reaction cavities 109 and, by bypassing the optional intermediate temperature-control cavity 110, also directly to the sensor apparatus 113, and/or in order for it to be possible to convey or pump liquids or liquid reagents F2-F5 out of the storage cavities 108B-108E into the sensor apparatus 113, in particular in the opposite direction to the analytes A and/or amplification products V, when the bypass 114A is open, more specifically when the valve 115B of the bypass 114A is open.

In particular, the cartridge 100 allows or provides a nucleic-acid sequence assay and/or - preferably by using the bypass 114 A to avoid e.g. PCR - a protein assay.

In case of a protein assay, the sample P or a portion thereof may be delivered to the sensor apparatus 113 without amplification or the like, so that proteins of the sample P can be bonded as analytes A to the catcher molecules M.

The cartridge 100 or the fluid system 103 or the channels 114 preferably comprise sensor portions 116 or other apparatuses for detecting liquid fronts and/or flows of fluid.

It is noted that various components, such as the channels 114, the valves 115, in particular the valves 115A that are initially closed and the valves 115B that are initially open, and the sensor portions 116 in Fig. 2 are, for reasons of clarity, only labelled in some cases, but the same symbols are used in Fig. 2 for each of these components.

The collection cavity 111 is preferably used for receiving excess or used reagents and liquids and volumes of the sample. It is preferably given appropriate large dimensions and/or is only provided with inputs or inlets, in particular such that liquids cannot be removed or pumped out again in the operating position.

The receiving cavity 104 preferably comprises a connection 104A for introducing the sample P. After the sample P is introduced into the receiving cavity 104, said cavity and/or the connection 104A is closed.

The cartridge 100 can then be inserted into the proposed analysis device 200 and/or received thereby, as shown in Fig. 1, in order to test the sample P. Alternatively, the sample P could also be fed in later.

Fig. 1 shows the analysis system 1 in a ready-to-use state for carrying out a test on the sample P received in the cartridge 100. In this state, the cartridge 100 is therefore linked to, received by and/or inserted into the analysis device 200.

In the following, some features and aspects of the analysis device 200 are first explained in greater detail. The features and aspects relating to said device are preferably also directly features and aspects of the proposed analysis system 1, in particular even without any further explicit explanation.

The analysis system 1 or analysis device 200 preferably comprises a mount or receptacle 201 for mounting and/or receiving the cartridge 100.

Preferably, the cartridge 100 is fluidically, in particular hydraulically, separated or isolated from the analysis device 200. In particular, the cartridge 100 forms a preferably independent and in particular closed fluidic and/or hydraulic system 103 for the sample P and the reagents and other liquids.

Preferably, the analysis device 200 is designed to actuate the pump apparatus 112 and/or valves 115, to have a thermal effect and/or to detect measured data, in particular by means of the sensor apparatus 113 and/or sensor portions 116.

The analysis system 1 or analysis device 200 preferably comprises a pump drive 202, the pump drive 202 in particular being designed for mechanically actuating the pump apparatus 112.

Preferably, a head of the pump drive 202 can be rotated in order to rotationally axially depress the preferably bead-like raised portion of the pump apparatus 112. Particularly preferably, the pump drive 202 and pump apparatus 112 together form a pump, in particular in the manner of a hose pump or peristaltic pump and/or a metering pump, for the fluid system 103 and/or the cartridge 100.

Particularly preferably, the pump is constructed as described in DE 10 2011 015 184 B4. However, other structural solutions are also possible.

Preferably, the capacity and/or discharge rate of the pump can be controlled and/or the conveying direction of the pump and/or pump drive 202 can be switched. Preferably, fluid can thus be pumped forwards or backwards as desired.

The analysis system 1 or analysis device 200 preferably comprises a connection apparatus 203 for in particular electrically and/or thermally connecting the cartridge 100 and/or the sensor apparatus 113.

As shown in Fig. 1, the connection apparatus 203 preferably comprises a plurality of electrical contact elements 203A, the cartridge 100, in particular the sensor apparatus 113, preferably being electrically connected or connectable to the analysis device 200 by the contact elements 203A.

The analysis system 1 or analysis device 200 preferably comprises one or more temperature-control apparatuses 204, in particular heating elements or Peltier elements, for temperature-controlling the cartridge 100 and/or having a thermal effect on the cartridge 100, in particular for heating and/or cooling.

Individual temperature-control apparatuses 204, some of these apparatuses or all of these apparatuses can preferably be positioned against the cartridge 100, the main body 101, the cover 102, the sensor apparatus 113 and/or individual cavities and/or can be thermally coupled thereto and/or can be integrated therein and/or in particular can be operated or controlled electrically by the analysis device 200. In the example shown, in particular the temperature-control apparatuses 204A, 204B and/or 204C are provided.

Preferably, the temperature-control apparatus 204A, referred to in the following as the reaction temperature-control apparatus 204A, is assigned to the reaction cavity 109 or to a plurality of reaction cavities 109, in particular in order for it to be possible to carry out one or more amplification reactions and/or PCRs therein.

The reaction cavities 109 are preferably temperature-controlled simultaneously and/or uniformly, in particular by means of one common reaction temperature-control apparatus 204A or two reaction temperature-control apparatuses 204A.

More particularly preferably, the reaction cavity/cavities 109 can be temperature-controlled from two different sides and/or by means of two or the reaction temperature-control apparatuses 204A that are preferably arranged on opposite sides.

Alternatively, for reaction cavities 109, each reaction cavity 109 can be temperature-controlled independently and/or individually.

The temperature-control apparatus 204B, referred to in the following as the intermediate temperature-control apparatus 204B, is preferably assigned to an intermediate temperature-control cavity 110 and/or is designed to temperature-control the intermediate temperature-control cavity 110 or a fluid located therein, in particular the amplification products V.

Particularly preferably, the intermediate temperature-control cavity 110 and/or temperature-control apparatus 204B is designed or intended to denature the sample P or analytes A and/or the amplification products V produced, and/or to divide any double-stranded analytes A or amplification products V into single strands and/or to counteract premature bonding and/or hybridising of the amplification products V, in particular by the addition of heat.

The temperature-control apparatus 204C, referred to in the following as the sensor temperature-control apparatus 204C, is in particular assigned to the sensor apparatus 113 and/or is designed to temperature-control fluids located in or on the sensor apparatus 113, in particular analytes A and/or amplification products V, reagents or the like, in a desired manner.

The analysis system 1 or analysis device 200 preferably comprises one or more actuators 205 for actuating the valves 115. Particularly preferably, different (types or groups of) actuators 205A and 205B are provided which are assigned to the different (types or groups of) valves 115A and 115B for actuating each of said valves, respectively.

The analysis system 1 or analysis device 200 preferably comprises one or more sensors 206. In particular, the sensors 206A are designed or intended to detect liquid fronts and/or flows of fluid in the fluid system 103. Particularly preferably, the sensors 206A are designed to measure or detect, for example optically and/or capacitively, a liquid front and/or the presence, the speed, the mass flow rate/volume flow rate, the temperature and/or another value of a fluid in a channel and/or a cavity, in particular in a respectively assigned sensor portion 116, which is in particular formed by a planar and/or widened channel portion of the fluid system 103.

Particularly preferably, the sensor portions 116 are each oriented and/or incorporated in the fluid system 103 and/or fluid flows against or through the sensor portions 116 such that, in the operating position of the cartridge 100, fluid flows through the sensor portions 116 in the vertical direction and/or from the bottom to the top, in order to make it possible or easier to reliably detect liquid.

Alternatively or additionally, the analysis device 200 preferably comprises (other or additional) sensors 206B for detecting the ambient temperature, internal temperature, atmospheric humidity, position, and/or alignment, for example by means of a GPS sensor, and/or the orientation and/or inclination of the analysis device 200 and/or the cartridge 100.

The analysis system 1 or analysis device 200 preferably comprises a control apparatus 207, in particular comprising an internal clock or time base for controlling the sequence of a test and/or for collecting, evaluating and/or outputting or providing measured values in particular from the sensor apparatus 113, and/or from test results and/or other data or values.

The control apparatus 207 preferably controls or feedback controls the pump drive 202, the temperature-control apparatuses 204 and/or actuators 205, in particular taking into account or depending on the desired test and/or measured values from the sensor apparatus 113 and/or sensors 206.

Generally, it is noted that the cartridge 100, the fluid system 103 and/or the conveying of fluid preferably do not operate on the basis of capillary forces, but at least essentially or primarily under the effects of gravity and/or the effect of the pump or pump apparatus 112.

In the operating position, the liquids from the respective cavities are preferably removed, in particular drawn out, via the outlet that is at the bottom in each case, it being possible for gas or air to flow and/or be pumped into the respective cavities via the inlet that is in particular at the top. In particular, relevant vacuums in the cavities can thus be prevented or at least minimised when conveying the liquids.

The flows of fluid are controlled in particular by accordingly activating the pump or pump apparatus 112 and actuating the valves 115.

Particularly preferably, the pump drive 202 comprises a stepper motor, or a drive calibrated in another way, such that desired metering can be achieved, at least in principle, by means of appropriate activation.

Additionally or alternatively, sensors 206A are preferably used to detect liquid fronts or flows of fluid, in particular in cooperation with the assigned sensor portions 116, in order to achieve the desired fluidic sequence and the desired metering by accordingly controlling the pump or pump apparatus 112 and accordingly activating the valves 115.

Optionally, the analysis system 1 or analysis device 200 comprises an input apparatus 208, such as a keyboard, a touch screen or the like, and/or a display apparatus 209, such as a screen.

The analysis system 1 or analysis device 200 preferably comprises at least one interface 210, for example for controlling, for communicating and/or for outputting measured data or test results and/or for linking to other devices, such as a printer, an external power supply, a smartphone, a computer net, a computer cloud, the internet, a server, or the like. This may in particular be a wired or wireless interface 210.

The analysis system 1 or analysis device 200 preferably comprises a power supply 211, preferably a battery or an accumulator, which is in particular integrated and/or externally connected or connectable.

Preferably, an integrated accumulator is provided as a power supply 211 and is (re)charged by an external charging device (not shown) via a connection 211A and/or is interchangeable.

The analysis system 1 or analysis device 200 preferably comprises a housing 212, all the components and/or some or all of the apparatuses preferably being integrated in the housing 212. Particularly preferably, the cartridge 100 can be inserted or slid into the housing 212, and/or can be received by the analysis device 200, through an opening 213 which can in particular be closed, such as a slot or the like.

The analysis system 1 or analysis device 200 is preferably portable or mobile. Particularly preferably, the analysis device 200 weighs less than 25 kg or 20 kg, particularly preferably less than 15 kg or 10 kg, in particular less than 9 kg or 6 kg.

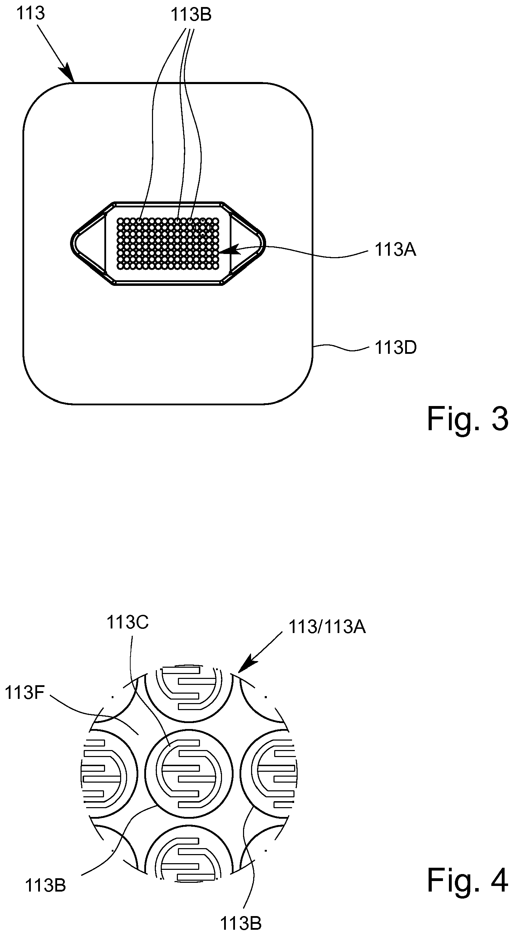

In the following, further details are given on a preferred construction of the sensor apparatus 113 with reference to Fig. 3 to Fig. 6.

The sensor apparatus 113 preferably allows electrochemical measurement and/or redoxcycling.

In particular, the sensor apparatus 113 is designed to identify, to detect and/or to determine (identical or different) analytes A bonded to capture molecules M or products derived therefrom, in particular amplification products V of the analyte A or different analytes A.

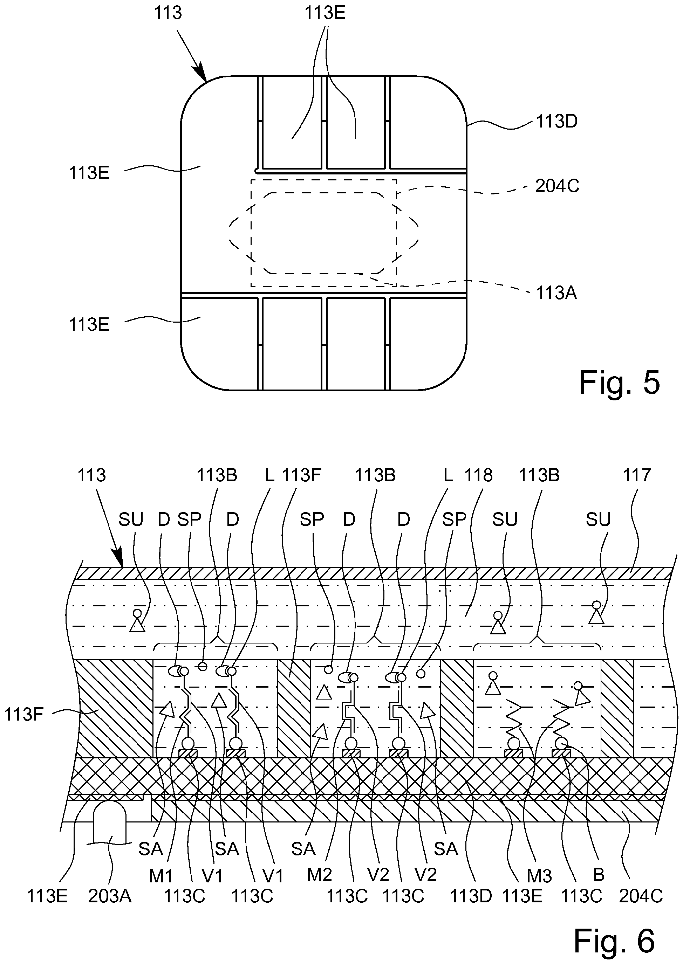

The sensor apparatus 113 preferably comprises a sensor array 113A comprising a plurality of sensor regions or sensor fields 113B, as shown schematically in Fig. 3, which schematically shows the measuring side of the sensor apparatus 113 and/or the sensor array 113A. Fig. 4 is an enlarged detail from Fig. 3. Fig. 5 shows a connection side and Fig. 6 is a schematic section through the sensor apparatus 113.

Preferably, the sensor apparatus 113 or the sensor array 113A comprises more than 10 or 20, particularly preferably more than 50 or 80, in particular more than 100 or 120 and/or less than 1000 or 800 sensor fields 113B.

Preferably, the sensor apparatus 113 or the sensor array 113A comprises a plurality of electrodes 113C. At least two electrodes 113C are preferably arranged in each sensor region or sensor field 113B. In particular, at least two electrodes 113C in each case form a sensor field 113B.

The electrodes 113C are preferably made of metal, in particular of noble metal, such as platinum or gold, and/or said electrodes are coated, in particular with thiols.

Preferably, the electrodes 113C are finger-like and/or engage in one another, as can be seen from the enlarged detail of a sensor field 113B according to Fig. 4. However, other structural solutions or arrangements are also possible.

The sensor apparatus 113 preferably comprises a support 113D, in particular a chip, the electrodes 113C preferably being arranged on the support 113D and/or being integrated in the support 113D.

The measuring side comprises the electrodes 113C and/or is the side that faces the fluid, the sample P, the amplification products V and/or a sensor compartment, and/or is the side of the sensor apparatus 113 and/or the support 113D comprising capture molecules M (as shown in Fig. 6) to which the analytes A and/or amplification products V are bonded.

The connection side of the sensor apparatus 113 and/or the support 113D is preferably opposite the measuring side and/or is the side that faces away from the fluid, the sample P and/or the amplification product V.

Particularly preferably, the measuring side and the connection side of the sensor apparatus 113 and/or the support 113D each form one flat side of the in particular planar and/or plate-like support 113D.

The sensor apparatus 113, in particular the support 113D, preferably comprises a plurality of, in this case eight, electrical contacts or contact surfaces 113E, the contacts 113E preferably being arranged on the connection side and/or forming the connection side, as shown in Fig. 5.

Preferably, the sensor apparatus 113 can be contacted on the connection side and/or by means of the contacts 113E and/or can be electrically connected to the analysis device 200. In particular, an electrical connection can be established between the cartridge 100, in particular the sensor apparatus 113, and the analysis device 200, in particular the control apparatus 207, by electrically connecting the contacts 113E to the contact elements 203A.

Preferably, the contacts 113E are arranged laterally, in the edge region and/or in a plan view or projection around the electrodes 113C and/or the sensor array 113A, and/or the contacts 113E extend as far as the edge region of the sensor apparatus 113, in particular such that the support 113D can be electrically contacted, preferably by means of the connection apparatus 203 or the contact elements 203A, as already explained, laterally, in the edge region and/or around the sensor temperature-control apparatus 204C, which can preferably be positioned centrally or in the middle on the support 113D.

Preferably, the sensor fields 113B are separated from one another, as shown in the schematic view from Fig. 6. In particular, the sensor apparatus 113 comprises barriers or partitions between each of the sensor fields 113B, which are preferably formed by an in particular hydrophobic layer 113F having corresponding recesses for the sensor fields 113B. However, other structural solutions are also possible.

The cartridge 100 and/or the sensor apparatus 113 comprises or forms a sensor compartment 118. In particular, the sensor compartment 118 is formed between the sensor array 113A, the sensor apparatus 113 and/or the support 113D, or between the measuring side on one side and a sensor cover 117 on the other side.

The sensor apparatus 113 preferably defines the sensor compartment 118 by means of its measuring side and/or the sensor array 113A. The electrodes 113C are therefore in the sensor compartment 118.

Preferably, the cartridge 100 and/or the sensor apparatus 113 comprises the sensor cover 117, the sensor compartment 118 in particular being defined or delimited by the sensor cover 117 on the flat side.

Particularly preferably, the sensor cover 117 can be lowered onto the partitions and/or layer 113F for the actual measurement.

The sensor apparatus 113 or the sensor compartment 118 is fluidically linked to the fluid system 103, in particular to the reaction cavity/cavities 109, preferably by connections, like an inlet 119 and an outlet 120, such that the (treated) sample P, the analytes A or amplification products V can be admitted to the measuring side of the sensor apparatus 113 or sensor array 113A.

The sensor compartment 118 can thus be loaded with fluids and/or said fluids can flow therethrough.

The sensor apparatus 113 preferably comprises a plurality of in particular different capture molecules M, different capture molecules M preferably being arranged and/or immobilised in the (same) sensor compartment 118 and/or in or on different sensor fields 113B and/or preferably being assigned to different sensor fields 113B.

Particularly preferably, the electrodes 113C are provided with capture molecules M, in this case via bonds B, in particular thiol bonds, in particular in order to bond and/or detect or identify suitable analytes A and/or amplification products V.

Different capture molecules M1 to M3 are preferably provided for the different sensor fields 113B and/or the different electrode pairs and/or electrodes 113C, in order to specifically bond different analytes A and/or amplification products V, in Fig. 6 the amplification products V1 to V3, in the sensor fields 113B.

Particularly preferably, the sensor apparatus 113 or sensor array 113A allows the amplification products V bonded in each sensor field 113B to be qualitatively or quantitatively determined.

Optionally, the sensor apparatus 113 comprises capture molecules M having different hybridisation temperatures, preferably in order to bond the amplification products V to the corresponding capture molecules M at different hybridisation temperatures.

Preferably, the different capture molecules M having different hybridisation temperatures are arranged or immobilized in or within the (same) sensor compartment 118 of the sensor apparatus 113. This allows in particular a very compact and simple realization and/or detection or identification of a multiplicity of analytes A, amplification products V and/or groups by means of or within one sensor apparatus 113 or sensor compartment 118.

Preferably, in the operating state, the sensor temperature-control apparatus 204C rests on the support 113D in a planar manner and/or centrally and/or so as to be opposite the sensor array 113A and/or rests on one or more contacts 113E at least in part. This makes it possible to particularly rapidly and efficiently temperature-control the sensor compartment 118 and/or amplification products V.

The sensor apparatus 113, in particular the support 113D, preferably comprises at least one, preferably a plurality of, electronic or integrated circuits, the circuits in particular being designed to detect electrical currents or voltages that are preferably generated at the sensor fields 113B in accordance with the redoxcycling principle.

Particularly preferably, the measurement signals from the different sensor fields 113B are separately collected or measured by the sensor apparatus 113 and/or the circuits.

Particularly preferably, the sensor apparatus 113 and/or the integrated circuits directly convert the measurement signals into digital signals or data, which can in particular be read out by the analysis device 200.

Particularly preferably, the sensor apparatus 113 and/or the support 113D is constructed as described in EP 1 636 599 B1.

The analysis system 1 or analysis device 200 is preferably adapted for detecting or identifying further analytes A, compounds and/or material characteristics of the sample P by optical measurement and/or without specific bonding. This additional functionality is also called "additional measurement".

Preferably, the analysis system 1 or analysis device 200 comprises a (further) measurement apparatus 250 for the additional measurement, i. e. for detecting or identifying further analytes, compounds and/or material characteristics of the sample P, in particular by optical measurement and/or without specific bonding to preferably immobilized catcher molecules M.

Preferably, the measurement apparatus 250 forms part of or is integrated into the analysis device 200 or its housing 212.

Preferably, the measurement apparatus 250 is for optical measurement and, in particular, comprises or is formed by a spectrometer. This applies in particular if the additional measurement is an optical measurement, in particular optical spectroscopy. However, the additional measurement can be any of kind of other measurements without specific bonding by capture molecules M and/or without electrochemical detection by redoxcycling, such as impedance measurement, mass spectroscopy or the like.

The following description focuses on an optical measurement as additional measurement. In particular, the optical measurement can be performed in the visible and/or invisible spectrum, wherein the invisible spectrum can include measurements in the infrared and/or ultraviolet range. However, the description and respective features apply preferably also or in a similar manner for other kinds of additional measurements.

In the preferred embodiments shown in Figs. 1 and 2, the cartridge 100 forms a sample carrier 160 for carrying or providing the sample P for the additional measurement, preferably wherein the cartridge 100 / sample carrier 160 comprises a measurement chamber 161 for the additional measurement.

The measurement chamber 161 is preferably separate from the sensor apparatus 113, sensor compartment 118 and/or reaction cavity 109.

Alternatively, the measurement chamber 161 is integrated into or formed by or within the sensor apparatus 113 and/or sensor compartment 118.

In the first embodiment shown in Fig. 1, the measurement chamber 161 is preferably formed by the receiving cavity 104 or vice versa.

The measurement chamber 161 can be formed separately from the receiving cavity 104 and/or by a separate or dedicated cavity as shown in Fig. 2 representing a slightly modified or other embodiment of the cartridge 100 with separate measurement chamber 161.

In the shown embodiment, the measurement chamber 161 is preferably fluidically connected with or to the receiving cavity 104, in particular via a connection channel 162 as schematically indicated in Fig. 2. Thus, the measurement chamber 161 could be filled simultaneously with or after the receiving cavity 104 and/or automatically when the sample P is filled into the cartridge 100. However, other constructional solutions are possible as well.

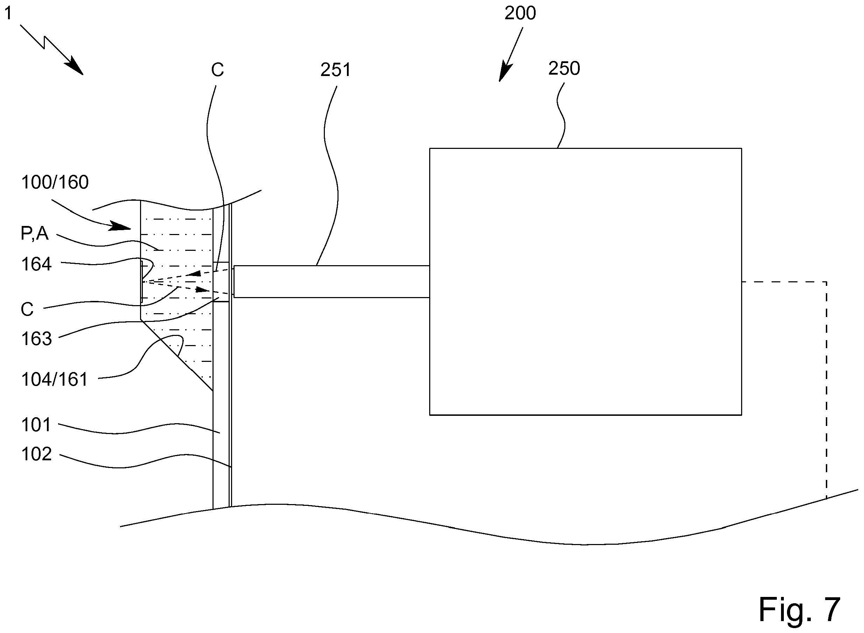

Fig. 7 shows a schematic, partial enlargement of Fig. 1 in the region of the receiving cavity 104 / measurement chamber 161.

The analysis system 1, analysis device 200 or measurement apparatus 250 may comprise a light guide 251 for guiding light C from the measurement apparatus 250 to or into the measurement chamber 161 or sample P and back from the measuring chamber 161 or sample P to the measurement apparatus 250. However, other constructional solutions are possible as well.

Preferably, the sample carrier 160 or measurement chamber 161 comprises an optical window 163 so that the light C can enter into the measurement chamber 161 or sample P contained therein and so that the light C can exit through the optical window 163 for returning to the light guide 251 and/or measurement apparatus 250 as shown schematically in Fig. 7.

The optical window 163 may be formed or covered by the film or cover 102 and/or any other transparent component, insert or the like.

The sample carrier 160 or measurement chamber 161 comprises preferably a reflector 164 for reflecting the light C and/or for optical measurements of the sample P in the measurement chamber 161. In particular, the reflector 164 reflects the light C back towards the optical window 163 and/or light guide 251 in the shown embodiment.

Preferably, the light C is reflected multiple times within the measurement chamber 162 before the light C returns back to a sensor, here the measurement apparatus 250.

It has to be noted that the light C can be emitted from a separate apparatus (not shown) and/or can enter the measurement chamber 161 or sample P differently through another pathway or optical window 163 than the optical window 163 or pathway to the light guide 251 and/or measurement apparatus 250.

A method according to the present invention provides in particular the additional measurement in addition to the primary measurement, wherein the primary measurement is or includes preferably a protein assay and/or a nucleic-acid sequence assay performed on or by the cartridge 100 or its sensor apparatus 113.

The additional measurement is preferably realized or conducted independently from the primary measurement on the cartridge 100 or by the cartridge 100.

In particular, the measurement apparatus 250 works preferably independently from the sensor apparatus 113 or vice versa.

It is possible that the additional measurement and the primary measurement (the measurement usually conducted on or by the cartridge 100 or sensor apparatus 113) are conducted simultaneously. However, it is also possible that the additional measurement is conducted at first and, then, the primary measurement is conducted. This may apply in particular when the additional measurement is conducted on the sample P in the measurement chamber 161 and, afterwards, the sample P is at least partially drained from the measurement chamber 161, e.g. from the receiving cavity 104, for the primary measurement.

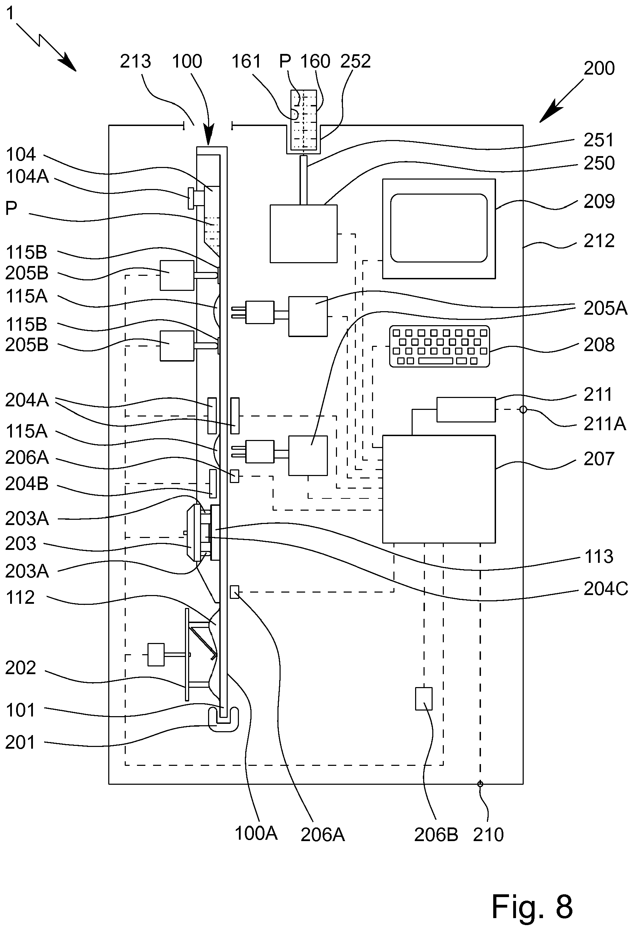

Alternatively or additionally, the sample P may be contained in a sample carrier 160 separate from the cartridge 100 for the additional measurement as schematically shown in Fig. 8 which shows in a similar schematic section as Fig. 1 another embodiment of the analysis system 1 and analysis device 200. Here, the analysis system 1 or analysis device 200 comprises preferably an interface 252 for connecting - in particular mechanically and/or optically - the sample carrier 160 to or with the analysis device 200 and/or measurement apparatus 250 or light guide 251.

The separate sample carrier 160 comprises or forms preferably as well a measurement chamber 161 and/or comprises preferably the optical window 163 and/or reflector 164 as already described with regard to the other embodiments.

In the embodiment with separate receptacle or interface 252 for the sample carrier 160 for the additional measurement, the additional measurement and the primary measurement can be realized and performed completely independently from each other.

According to the present invention, the primary measurement, in particular such as a protein assay and/or nucleic-acid assay and/or any other assay where analytes A or amplifications V, proteins or the like are bonded by catcher molecules M (which is preferably realized in or on or by the cartridge 100 or sensor apparatus 113), is combined with the at least one additional measurement as outlined above. It is even possible to combine the primary measurement with multiple additional measurements, in particular additional measurements of different kind.

Preferably, the primary measurement in form of an electrochemical measurement, in particular including redoxcycling, is combined with the additional measurement in form of an optical measurement according to the present invention.

Preferably, the primary measurement including specific bonding to catcher molecules or the like is combined with the additional measurement in form of a non-bonding measurement according to the present invention.

Preferably, the primary measurement is conducted on or in the cartridge 100 or sensor apparatus 113, while the additional measurement is conducted or at least measured outside the cartridge 100 or sensor apparatus 113, preferably by the analysis device 200 or its measurement apparatus 250.

In the following, a preferred sequence of a test or analysis using the proposed analysis system 1 and/or analysis device 200 and/or the proposed cartridge 100 is explained in greater detail as an example for a primary measurement according to the present invention.

The analysis system 1, the cartridge 100 and/or the analysis device 200 is preferably designed to carry out the proposed method.

During the proposed method for testing a sample P, at least one analyte A of the sample P is preferably amplified or copied, in particular by means of PCR. The amplified analyte A and/or the amplification products V produced in this way is/are then bonded and/or hybridised to corresponding capture molecules M. The bonded amplification products V are then detected, in particular by means of electronic measurement.

The method may be used in particular in the field of medicine, in particular veterinary medicine, in order to detect diseases and/or pathogens.

Within the context of the method according to the invention, a sample P having at least one analyte A on the basis of a fluid or a liquid from the human or animal body, in particular blood, saliva or urine, is usually first introduced into the receiving cavity 104 via the connection 104A, in order to detect diseases and/or pathogens, it being possible for the sample P to be pretreated.

Once the sample P has been received, the receiving cavity 104 and/or the connection 104A thereof is fluidically closed, in particular in a liquid-tight and/or gas-tight manner.

Preferably, the cartridge 100 together with the sample P is then linked or connected to the analysis device 200, in particular is inserted or slid into the analysis device 200.

The method sequence, in particular the flow and conveying of the fluids, the mixing and the like, is controlled by the analysis device 200 or the control apparatus 207, in particular by accordingly activating and actuating the pump drive 202 or the pump apparatus 112 and/or the actuators 205 or valves 115.

Preferably, the sample P, or some of or a supernatant of the sample P, is removed from the receiving cavity 104 via the outlet 104C and/or the intermediate connection 104D and is fed to the mixing cavity 107 in a metered manner.

Preferably, the sample P in the cartridge 100 is metered, in particular in or by means of the first metering cavity 105A and/or second metering cavity 105B, before being introduced into the mixing cavity 107. Here, in particular the upstream and/or downstream sensor portions 116 are used together with the assigned sensors 206 in order to make possible the desired metering. However, other solutions are also possible.

In the mixing cavity 107, the sample P is prepared for further analysis and/or is mixed with a reagent, preferably with a liquid reagent F1 from a first storage cavity 108A and/or with one or more dry reagents S1, S2 and/or S3, which are preferably provided in the mixing cavity 107.

The liquid and/or dry reagents can be introduced into the mixing cavity 107 before and/or after the sample P. In the example shown, the dry reagents S1 to S3 are preferably introduced into the mixing cavity 107 previously and are optionally dissolved by the sample P and/or the liquid reagent F1.

The liquid reagent F1 may in particular be a reagent, in particular a PCR master mix, for the amplification reaction or PCR. Preferably, the PCR master mix contains nuclease-free water, enzymes for carrying out the PCR, in particular at least one DNA polymerase, nucleoside triphosphates (NTPs), in particular deoxynucleotides (dNTPs), salts, in particular magnesium chloride, and/or reaction buffers.

The dry reagents S1, S2 and/or S3 may likewise be reagents required for carrying out an amplification reaction or PCR, which are in a dry, in particular lyophilised, form. Preferably, the dry reagents S1, S2 and/or S3 are selected in particular from lyophilised enzymes, preferably DNA polymerases, NTPs, dNTPs and/or salts, preferably magnesium chloride.

The dissolving or mixing in the mixing cavity 107 takes place or is assisted in particular by introducing and/or blowing in gas or air, in particular from the bottom. This is carried out in particular by accordingly pumping gas or air in the circuit by means of the pump or pump apparatus 112.

Subsequently, a desired volume of the sample P that is mixed and/or pretreated in the mixing cavity 107 is preferably fed to one or more reaction cavities 109, particularly preferably via (respectively) one of the upstream, optional intermediate cavities 106A to 106C and/or with different reagents or primers, in this case dry reagents S4 to S6, being added or dissolved.

Particularly preferably, the (premixed) sample P is split into several sample portions, preferably of equal size, and/or is divided between the intermediate cavities 106A to 106C and/or reaction cavities 109, preferably evenly and/or in sample portions of equal size.

Different reagents, in the present case dry reagents S4 to S6, particularly preferably primers, in particular those required for the PCR or PCRs, in particular groups of different primers in this case, are preferably added to the (premixed) sample P in the intermediate cavities 106A to 106C and/or different reaction cavities 109, respectively.

The primers in the different groups differ in particular in terms of the hybridisation temperatures of the amplification products V produced by the respective primers. As a result, in particular the different group temperatures of the groups of analytes A and/or amplification products V are produced, as already mentioned at the outset.

Particularly preferably, marker primers are used in the sense already specified at the outset.

In the embodiment shown, the reagents or primers S4 to S6 are contained in the intermediate cavities 106A to 106C. However, other solutions are also possible, in particular those in which the reagents or primers S4 to S6 are contained in the reaction cavities 109.

According to a preferred embodiment, the intermediate cavities 106A to 106C each contain primers for amplifying/copying one analyte A, preferably two different analytes A and more preferably three different analytes A. However, it is also possible for four or more different analytes A to be amplified/copied per reaction cavity 109.

Particularly preferably, the reaction cavities 109 are filled in succession with a specified volume of the (pretreated) sample P or with respective sample portions via the intermediate cavities 106A to 106C that are each arranged upstream. For example, the first reaction cavity 109A is filled with a specified volume of the pretreated sample P before the second reaction cavity 109B and/or the second reaction cavity 109B is filled therewith before the third reaction cavity 109C.

In the reaction cavities 109, the amplification reactions or PCRs are carried out to copy/amplify the analytes A. This is carried out in particular by means of the assigned, preferably common, reaction temperature-control apparatus(es) 204A and/or preferably simultaneously for all the reaction cavities 109, i.e. in particular using the same cycles and/or temperature (curves/profiles).

The PCR or PCRs are carried out on the basis of protocols or temperature profiles that are essentially known to a person skilled in the art. In particular, the mixture or sample volume located in the reaction cavities 109 is preferably cyclically heated and cooled.

Preferably, nucleic-acid products are produced from the analytes A as amplification products V in the reaction cavity/cavities 109.

During the pretreatment, reaction and/or PCR or amplification, a label L is directly produced (in each case) and/or is attached to the amplification products V. This is in particular achieved by using corresponding, preferably biotinylated, primers. However, the label L can also be produced and/or bonded to the amplification products V separately or later, optionally also only in the sensor compartment 118 and/or after hybridisation.

The label L is used in particular for detecting bonded amplification products V. In particular, the label L can be detected or the label L can be identified in a detection process, as explained in greater detail in the following.

According to the invention, it is possible for a plurality of amplification reactions or PCRs to be carried out in parallel and/or independently from one another using different primers S4 to S6 and/or primer pairs, such that a large number of (different) analytes A can be copied or amplified in parallel and subsequently analysed.

In particular, identical or different analytes A1 are amplified in the first reaction cavity 109A, identical or different analytes A2 are amplified in the second reaction cavity 109B and identical or different analytes A3 are amplified in the third reaction cavity 109C, preferably by means of amplification reactions, in particular PCRs, that run in parallel.

Particularly preferably, the analytes A1 to A3 are different from one another, in particular such that a large number of different analytes A can be amplified and/or tested by means of the method. Preferably, more than 2 or 4, particularly preferably more than 8 or 11, in particular more than 14 or 17, analytes A can be tested and/or amplified, in particular at the same time.

In particular, a plurality of groups of amplification products V of the analytes A are formed and/or produced, preferably in parallel and/or independently from one another and/or in the reaction cavities 109. Therefore, for example, a first group of amplification products V1 of the analytes A1 is formed and/or produced in the first reaction cavity 109A, a second group of amplification products V2 of the analytes A2 is formed and/or produced in the second reaction cavity 109B, and a third group of amplification products V3 of the analytes A3 is formed and/or produced in the optional third reaction cavity 109C.

Particularly preferably, groups of (amplified) analytes A and/or amplification products V are formed that have different group temperatures in the sense mentioned at the outset. The groups thus preferably have different (optimal) hybridisation temperatures TH and/or ranges of hybridisation temperatures.

Preferably, different groups of analytes A and/or amplification products V, i.e. in particular nucleic-acid products and/or sequences, are thus amplified and/or formed for the test, it being possible, for the different groups to be amplified and/or formed and/or provided in particular in the different reaction chambers 109A to 109C, but alternatively also in a different manner.

After carrying out the PCR and/or amplification, corresponding fluid volumes and/or amplification products V and/or the groups are conducted out of the reaction cavities 109 in succession to the sensor apparatus 113 and/or to the sensor compartment 118, in particular via a group-specific and/or separate intermediate cavity 106E, 106F or 106G (respectively) and/or via the optional (common) intermediate temperature-control cavity 110.

The intermediate cavities 106E to 106G may contain further reagents, in this case dry reagents S9 and S10, respectively, for preparing the amplification products V for the hybridisation, e.g. a buffer, in particular an SSC buffer, and/or salts for further conditioning. On this basis, further conditioning of the amplification products V can be carried out, in particular in order to improve the efficiency of the subsequent hybridisation (bonding to the capture molecules M). Particularly preferably, the pH of the sample P is set or optimised in the intermediate cavities 106E to 106G and/or by means of the dry reagents S9 and S10.

Preferably, the sample P or the analytes A and/or amplification products V or groups formed thereby is/are, in particular immediately before being fed to the sensor apparatus 113 and/or between the reaction cavities 109 and the sensor apparatus 113, actively temperature-controlled (in particular in advance and/or before being temperature-controlled in the sensor apparatus 113), preferably preheated, in particular by means of and/or in the intermediate temperature-control cavity 110 and/or by means of the intermediate temperature-control apparatus 204B.

Preferably, the groups and/or analytes A or amplification products V of the individual reaction cavities 109 are actively temperature-controlled (in particular in advance and/or before being temperature-controlled in the sensor apparatus 113) and/or fed to the intermediate temperature-control cavity 110 in succession. The groups are in particular fed to the sensor apparatus 113 and/or the sensor compartment 118 in succession being temperature-controlled, in particular in advance and/or before being temperature-controlled in the sensor apparatus 113.

Once the sample P, groups, analytes A and/or amplification products V are hybridised and/or bonded to the capture molecules M, detection follows, in particular by means of the preferably provided labels L, or in another manner.

In the following, a particularly preferred variant of the detection is described in greater detail, specifically electrochemical detection, but other types of detection, for example optical detection, capacitive detection or the like, may also be carried out.

Following the respective bondings/hybridisations, preferably an optional washing process takes place and/or additional reagents or liquids, in particular from the storage cavities 108B to 108E, are optionally fed in.

In particular, it may be provided that sample residues and/or unbonded amplification products V, reagents and/or remnants of the PCR and other substances that may disrupt the rest of the method sequence are removed.

Washing or flushing may in particular take place using a fluid and/or reagent F3, in particular a wash buffer, particularly preferably a sodium-citrate buffer or SSC buffer, which is preferably contained in the storage cavity 108C. Unbonded analytes A and/or amplification products V, and substances which could disrupt subsequent detection, are preferably removed from the sensor apparatus 113 and/or fed to the collection cavity 111 by the wash buffer.

Subsequently and/or after the washing process, in accordance with a preferred variant of the method, detection of the amplification products V bonded to the capture molecules M takes place.

In order to detect the amplification products V bonded to the capture molecules M, a reagent F4 and/or detector molecules D, in particular alkaline phosphatase/streptavidin, is/are fed to the sensor apparatus 113, preferably from the storage cavity 108D.

The reagents F4 and/or detector molecules D can bond to the bonded amplification products V, in particular to the label L of the bonded amplification products V, particularly preferably to the biotin marker, as shown in Fig. 6.

In the context of detection, it may also be provided that additional liquid reagents F3 and/or F5 are fed from the storage cavities 108C and/or 108E to the sensor apparatus 113.

Optionally, subsequently or after the reagents F4 and/or detector molecules D have bonded to the amplification products V and/or the labels L, an (additional) washing process and/or flushing takes place, preferably by means of the fluid and/or reagent F3 and/or wash buffer, in particular in order to remove unbonded reagents F4 and/or detector molecules D from the sensor apparatus 113.

Preferably, a reagent S7 and/or S8 and/or substrate SU for the detection, in particular from the storage cavity 106D, is lastly fed to the sensor apparatus 113, preferably together with a fluid or reagent F2 (in particular a buffer), which is suitable for the substrate SU, particularly preferably for dissolving the reagent S7 and/or S8 and/or substrate SU, the fluid or reagent F2 in particular being taken from the storage cavity 106B. In particular, the reagent S7 and/or S8 can form or can comprise the substrate SU.

After adding the substrate SU, the sensor cover 117 is preferably lowered in order to isolate the sensor fields 113B from one another and/or to minimise the exchange of substances therebetween.

Preferably, p-aminophenyl phosphate (pAPP) is used as the substrate SU.

The substrate SU preferably reacts on and/or with the bonded amplification products V and/or detector molecules D and/or allows these to be electrochemically measured.

Preferably, the substrate SU is split by the bonded detector molecules D, in particular the alkaline phosphatase of the bonded detector molecules D, preferably into a first substance SA, such as p-aminophenol, which is in particular electrochemically active and/or redox active, and a second substance SP, such as phosphate.

Preferably, the first or electrochemically active substance SA is detected in the sensor apparatus 113 or in the individual sensor fields 113B by electrochemical measurement and/or redoxcycling.

Particularly preferably, by means of the first substance SA, specifically a redox reaction takes place at the electrodes 113C, the first substance SA preferably discharging electrons to or receiving electrons from the electrodes 113C.

In particular, the presence of the first substance SA and/or the respective amounts in the respective sensor fields 113B is detected by the associated redox reactions. In this way, it can be determined qualitatively and in particular also quantitatively whether and how many analytes A and/or amplification products V are bonded to the capture molecules M in the respective sensor fields 113B. This accordingly gives information on which analytes A are or were present in the sample P, and in particular also gives information on the quantity of said analytes A.

In particular, by means of the redox reaction with the first substance SA, an electrical current signal or power signal is generated at the assigned electrodes 113C, the current signal or power signal preferably being detected by means of an assigned electronic circuit.

Depending on the current signal or power signal from the electrodes 113C that is generated in this way, it is determined whether and/or where hybridisation to the capture molecules M has occurred.

The measurement is preferably taken just once and/or for the entire sensor array 113A and/or for all the sensor fields 113B, in particular simultaneously or in parallel. In particular, the bonded groups and/or amplification products V from all the groups and/or reaction cavities 109 are detected, identified or determined simultaneously or in parallel in a single or common detection process.

In other words, the amplification products V from the individual reaction cavities 109 that are bonded at different and/or specifically selected hybridisation temperatures TH are detected together and/or in parallel, such that rapid measurement is possible, and high specificity in relation to the hybridisation of the analytes A and/or amplification products V to the capture molecules M is nevertheless also achieved on the basis of the hybridisation temperature TH that is set in a targeted manner in each case.

However, in principle, it is also possible to measure a plurality of sample portions in the sensor apparatus 113 or in a plurality of sensor apparatuses 113 in succession or separately.

The test results or measurement results of the primary measurement and/or additional measurement are in particular electrically transmitted to the analysis device 200 or the control apparatus 207 thereof and are accordingly prepared, analysed, stored, displayed and/or output, in particular by the display apparatus 209 and/or interface 210.

In particular, the measurement results may be transmitted by wire or wirelessly to another device, computer, server, or the like, in particular for analysing, evaluating and/or further processing.

It has to be noted that "additional measurement" does not mean only one measurement, but can include also multiple measurements.

After the test has been carried out, the cartridge 100 and/or sample carrier 160 may be disconnected from the analysis device 200 and/or is released or ejected therefrom and are in particular disposed of.

Individual aspects and features of the present disclosure and individual method steps and/or method variants may be implemented independently from one another, but also in any desired combination and/or order.

### List of reference signs:

- 1: analysis system

- 100: cartridge
- 100A: front
- 101: main body
- 102: cover
- 103: fluid system
- 104: receiving cavity
- 104A: connection
- 104B: inlet
- 104C: outlet
- 104D: intermediate connection
- 105: metering cavity
- 105A: first metering cavity
- 105B: second metering cavity
- 106(A-G): intermediate cavity
- 107: mixing cavity
- 108(A-E): storage cavity
- 109: reaction cavity
- 109A: first reaction cavity
- 109B: second reaction cavity
- 109C: third reaction cavity
- 110: intermediate temperature control cavity
- 111: collection cavity
- 112: pump apparatus
- 113: sensor apparatus
- 113A: sensor array
- 113B: sensor field
- 113C: electrode
- 113D: support
- 113E: contact
- 113F: layer
- 114: channel
- 114A: bypass
- 115: valve
- 115A: initially closed valve
- 115B: initially open valve
- 116: sensor portion
- 117: sensor cover
- 118: sensor compartment
- 119: inlet
- 120: outlet
- 160: sample carrier
- 161: measurement chamber
- 162: connection channel
- 163: optical window
- 164: reflector

- 200: analysis device
- 201: receptacle
- 202: pump drive
- 203: connection apparatus
- 203A: contact element
- 204: temperature-control apparatus
- 204A: reaction temperature-control apparatus
- 204B: intermediate temperature-control apparatus
- 204C: sensor temperature-control apparatus
- 205: (valve) actuator
- 205A: (valve) actuator for 115A
- 205B: (valve) actuator for 115B
- 206: sensor
- 206A: fluid sensor
- 206B: other sensor
- 207: control apparatus
- 208: input apparatus
- 209: display apparatus
- 210: interface
- 211: power supply
- 211A: connection
- 212: housing
- 213: opening
- 250: measurement apparatus
- 251: light guide
- 252: interface

- A(1-3): analyte
- B: bond
- C: light
- D: detector molecule
- F(1-5): liquid reagent
- L: label
- M(1-3): capture molecule
- P: sample
- S(1-10): dry reagent
- SA: first substance
- SP: second substance
- SU: substrate

## Claims

1. Analysis system (1) for testing an in particular biological sample (P),
the analysis system (1) being adapted for electrochemically detecting or identifying one or more analytes (A) of the sample (P) and/or for detecting or identifying one or more analytes (A) of the sample (P) by specifically bonding the analytes (A) to one or more preferably immobilized catcher molecules (M) as primary measurement,
wherein the analysis system (1) is adapted for detecting or identifying further analytes (A), compounds and/or material characteristics of the sample (P) by optical measurement, preferably without specific bonding, of the sample (P) as additional measurement,
wherein the analysis system (1) is mobile and comprises a cartridge (100) for receiving the sample (P) and an analysis device (200) for connecting or receiving the cartridge (100) with the sample (P),
wherein the cartridge (100) comprises a fluid system (103) having a plurality of channels (114), cavities (104-111) and valves (115) for controlling the flow through the channels and/or cavities (104-111),
wherein the cartridge (100) comprises a sensor apparatus (113) for electrochemically detecting or identifying the one or more analytes (A) of the sample (P) and/or for detecting or identifying one or more analytes (A) of the sample (P) by specifically bonding the analyte or analytes (A) to one or more catcher molecules (M),
wherein the analysis device (200) is adapted to control the testing of the sample (P) in the cartridge (100) and to actuate the valves (115), and
wherein the analysis device (200) comprises a spectrometer for the additional measurement working independently from the sensor apparatus (113) for the primary measurement.

2. Analysis system according to claim 1, **characterised in that** the analysis device (200) comprises a receptacle (201) for receiving or connecting the cartridge (100) and in particular a connection apparatus (203) for electrically connecting the cartridge (100) or the sensor apparatus (113) thereon.

3. Analysis system according to claim 1 or 2, **characterised in that** the analysis device (200) comprises an interface (252) for connecting a sample carrier (160) for detecting or identifying the further analytes, compounds and/or material characteristics of the sample (P) by optical measurement and/or without specific bonding.

4. Analysis system according to any one of the preceding claims, **characterised in that** the cartridge (100) comprises a measurement chamber (161) for receiving the sample (P) for the additional measurement.

5. Analysis system according to claim 4, wherein the measurement chamber (161) is a receiving cavity (104) of the cartridge (100) for receiving the sample (P), the receiving cavity (104) comprising a closable connection (104A) for introducing the sample (P) into the receiving cavity (104).

6. Analysis system according to claim 4 or 5, **characterised in that** the measurement chamber (161) comprises an optical window (163) and a reflector (164) for optical measurements of the sample (P) in the measurement chamber (161).

7. Analysis system according to any one of claims 4 to 6, **characterised in that** the cartridge (100) comprises a reaction cavity (109) for PCR amplification, and **in that** the measurement chamber (161) is separate and/or distant from the reaction cavity (109).

8. Analysis system according to any one of claims 4 to 7, **characterised in that** the measurement chamber (161) is separate from the sensor apparatus (113).

9. Analysis system according to any one of the claims 1 to 3, **characterised in that** the analysis system (1) comprises a sample carrier (160) for the additional measurement, wherein the sample carrier (160) is separate from the cartridge (100).

10. Analysis system according to any one of the preceding claims, **characterised in that** the cartridge (100) or sensor apparatus (113) is designed to perform a nucleic-acid sequence assay and/or a protein assay.

11. Method for testing an in particular biological sample (P),
wherein a mobile analysis device (200) connects or receives a cartridge (100) with the sample (P),
wherein the analysis device (200) controls the testing of the sample (P) in the cartridge (100) and actuates valves (115) of the cartridge (100) for controlling the flow through channels (114) and/or cavities (104-111) of the cartridge (100),
wherein a sensor apparatus (113) of the cartridge (100) detects or identifies one or more analytes (A) of the sample (P) by specifically bonding the analyte(s) (A) to one or more catcher molecules (M) and/or by electrochemically measurement or redoxcycling,
wherein the analysis device (200) detects or identifies further analytes (A), compounds and/or material characteristics of the sample (P) by optical measurement, preferably without specific bonding, by means of a spectrometer of the analysis device (200) working independently from the sensor apparatus (113).

## Patentansprüche

1. Analysesystem (1) zur Untersuchung einer insbesondere biologischen Probe (P),
wobei das Analysesystem (1) dazu ausgebildet ist, einen oder mehrere Analyten (A) der Probe (P) elektrochemisch nachzuweisen oder zu identifizieren und/oder einen oder mehrere Analyten (A) der Probe (P) durch spezifisches Binden der Analyten (A) an ein oder mehrere vorzugsweise immobilisierte Fängermoleküle (M) als primäre Messung nachzuweisen oder zu identifizieren,
wobei das Analysesystem (1) dazu ausgebildet ist, weitere Analyten (A), Verbindungen und/oder Materialeigenschaften der Probe (P) durch optische Messung, vorzugsweise ohne spezifisches Binden, der Probe (P) als zusätzliche Messung nachzuweisen oder zu identifizieren,
wobei das Analysesystem (1) mobil ist und eine Cartridge (100) zum Aufnehmen der Probe (P) und ein Analysegerät (200) zum Anschließen oder Aufnehmen der Cartridge (100) mit der Probe (P) aufweist,
wobei die Cartridge (100) ein Fluidsystem (103) mit einer Vielzahl von Kanälen (114), Kavitäten (104-111) und Ventilen (115) zum Steuern des Flusses durch die Kanäle und/oder Kavitäten (104-111) aufweist,
wobei die Cartridge (100) eine Sensoreinrichtung (113) zum elektrochemischen Nachweisen oder Identifizieren des einen oder der mehreren Analyten (A) der Probe (P) und/oder zum Nachweisen oder Identifizieren eines oder mehrerer Analyten (A) der Probe (P) durch spezifisches Binden des oder der Analyten (A) an ein oder mehrere Fängermoleküle (M) aufweist,
wobei das Analysegerät (200) dazu ausgebildet ist, die Untersuchung der Probe (P) in der Cartridge (100) zu steuern und die Ventile (115) zu betätigen, und
wobei das Analysegerät (200) ein Spektrometer für die zusätzliche Messung aufweist, das unabhängig von der Sensoreinrichtung (113) für die primäre Messung arbeitet.

2. Analysesystem nach Anspruch 1, **dadurch gekennzeichnet, dass** das Analysegerät (200) eine Aufnahme (201) zum Aufnehmen oder Anschließen der Cartridge (100) und insbesondere eine Anschlusseinrichtung (203) zum elektrischen Anschließen der Cartridge (100) oder der daran befindlichen Sensoreinrichtung (113) aufweist.

3. Analysesystem nach Anspruch 1 oder 2, **dadurch gekennzeichnet, dass** das Analysegerät (200) eine Schnittstelle (252) zum Anschließen eines Probenträgers (160) zum Nachweisen oder Identifizieren der weiteren Analyten, Verbindungen und/oder Materialeigenschaften der Probe (P) durch optische Messung und/oder ohne spezifisches Binden aufweist.

4. Analysesystem nach einem der voranstehenden Ansprüche, **dadurch gekennzeichnet, dass** die Cartridge (100) eine Messkammer (161) zum Aufnehmen der Probe (P) für die zusätzliche Messung aufweist.

5. Analysesystem nach Anspruch 4, wobei die Messkammer (161) eine Aufnahmekavität (104) der Cartridge (100) zur Aufnahme der Probe (P) ist, wobei die Aufnahmekavität (104) einen verschließbaren Anschluss (104A) zum Einbringen der Probe (P) in die Aufnahmekavität (104) aufweist.

6. Analysesystem nach Anspruch 4 oder 5, **dadurch gekennzeichnet, dass** die Messkammer (161) ein optisches Fenster (163) und einen Reflektor (164) für optische Messungen der Probe (P) in der Messkammer (161) aufweist.

7. Analysesystem nach einem der Ansprüche 4 bis 6, **dadurch gekennzeichnet, dass** die Cartridge (100) eine Reaktionskavität (109) zur PCR-Amplifikation aufweist, und dass die Messkammer (161) von der Reaktionskavität (109) getrennt und/oder beabstandet ist.

8. Analysesystem nach einem der Ansprüche 4 bis 7, **dadurch gekennzeichnet, dass** die Messkammer (161) von der Sensoreinrichtung (113) getrennt ist.

9. Analysesystem nach einem der Ansprüche 1 bis 3, **dadurch gekennzeichnet, dass** das Analysesystem (1) einen Probenträger (160) für die zusätzliche Messung aufweist, wobei der Probenträger (160) von der Cartridge (100) getrennt ist.

10. Analysesystem nach einem der voranstehenden Ansprüche, **dadurch gekennzeichnet, dass** die Cartridge (100) oder die Sensoreinrichtung (113) zur Durchführung eines Nukleinsäuresequenz-Assays und/oder eines Protein-Assays ausgebildet ist.

11. Verfahren zur Untersuchung einer insbesondere biologischen Probe (P),
wobei ein mobiles Analysegerät (200) eine Cartridge (100) mit der Probe (P) verbindet oder aufnimmt,
wobei das Analysegerät (200) die Untersuchung der Probe (P) in der Cartridge (100) steuert und Ventile (115) der Cartridge (100) zum Steuern des Flusses durch Kanäle (114) und/oder Kavitäten (104-111) der Cartridge (100) betätigt,
wobei eine Sensoreinrichtung (113) der Cartridge (100) einen oder mehrere Analyten (A) der Probe (P) durch spezifisches Binden des/der Analyten (A) an ein oder mehrere Fängermoleküle (M) und/oder durch elektrochemische Messung oder Redoxcycling nachweist oder identifiziert,
wobei das Analysegerät (200) weitere Analyten (A), Verbindungen und/oder Materialeigenschaften der Probe (P) durch optische Messung, vorzugsweise ohne spezifisches Binden, mittels eines unabhängig von der Sensoreinrichtung (113) arbeitenden Spektrometers des Analysegeräts (200) nachweist oder identifiziert.

## Revendications

1. Système d'analyse (1) pour tester un échantillon (P), en particulier un échantillon biologique,
le système d'analyse (1) étant adapté pour détecter ou identifier électrochimiquement un ou plusieurs analytes (A) de l'échantillon (P) et/ou pour détecter ou identifier un ou plusieurs analytes (A) de l'échantillon (P) en liant spécifiquement les analytes (A) à une ou plusieurs molécules de capture (M) de préférence immobilisées en tant que mesure primaire,
le système d'analyse (1) étant adapté pour détecter ou identifier d'autres analytes (A), composés et/ou caractéristiques matérielles de l'échantillon (P) par mesure optique, de préférence sans liaison spécifique, de l'échantillon (P) en tant que mesure supplémentaire,
le système d'analyse (1) étant mobile et comprenant une cartouche (100) pour recevoir l'échantillon (P) et un dispositif d'analyse (200) pour connecter ou recevoir la cartouche (100) avec l'échantillon (P),
la cartouche (100) comprenant un système de fluide (103) ayant une pluralité de canaux (114), de cavités (104-111) et de vannes (115) pour contrôler l'écoulement à travers les canaux et/ou les cavités (104-111),
la cartouche (100) comprenant un appareil capteur (113) pour détecter ou identifier électrochimiquement le ou les analytes (A) de l'échantillon (P) et/ou pour détecter ou identifier un ou les analytes (A) de l'échantillon (P) en liant spécifiquement l'analyte ou les analytes (A) à une ou plusieurs molécules de capture (M),
le dispositif d'analyse (200) étant adapté pour commander le test de l'échantillon (P) dans la cartouche (100) et pour actionner les vannes (115), et
le dispositif d'analyse (200) comprenant un spectromètre pour la mesure supplémentaire fonctionnant indépendamment de l'appareil capteur (113) pour la mesure primaire.

2. Système d'analyse selon la revendication 1, **caractérisé en ce que** le dispositif d'analyse (200) comprend un réceptacle (201) pour recevoir ou connecter la cartouche (100) et en particulier un appareil de connexion (203) pour connecter électriquement la cartouche (100) ou l'appareil capteur (113) sur celle-ci.

3. Système d'analyse selon la revendication 1 ou 2, **caractérisé en ce que** le dispositif d'analyse (200) comprend une interface (252) pour connecter un support d'échantillon (160) pour détecter ou identifier les autres analytes, composés et/ou caractéristiques matérielles de l'échantillon (P) par mesure optique et/ou sans liaison spécifique.

4. Système d'analyse selon l'une quelconque des revendications précédentes, **caractérisé en ce que** la cartouche (100) comprend une chambre de mesure (161) destinée à recevoir l'échantillon (P) pour la mesure supplémentaire.

5. Système d'analyse selon la revendication 4, dans lequel la chambre de mesure (161) est une cavité de réception (104) de la cartouche (100) pour recevoir l'échantillon (P), la cavité de réception (104) comprenant une connexion (104A) pouvant être fermée pour introduire l'échantillon (P) dans la cavité de réception (104).

6. Système d'analyse selon la revendication 4 ou 5, **caractérisé en ce que** la chambre de mesure (161) comprend une fenêtre optique (163) et un réflecteur (164) pour les mesures optiques de l'échantillon (P) dans la chambre de mesure (161).

7. Système d'analyse selon l'une quelconque des revendications 4 à 6, **caractérisé en ce que** la cartouche (100) comprend une cavité de réaction (109) pour l'amplification PCR, et **en ce que** la chambre de mesure (161) est séparée et/ou distante de la cavité réaction (109).

8. Système d'analyse selon l'une quelconque des revendications 4 à 7, **caractérisé en ce que** la chambre de mesure (161) est séparée de l'appareil capteur (113).

9. Système d'analyse selon l'une quelconque des revendications 1 à 3, **caractérisé en ce que** le système d'analyse (1) comprend un support d'échantillon (160) pour la mesure supplémentaire, le support d'échantillon (160) étant séparé de la cartouche (100).

10. Système d'analyse selon l'une quelconque des revendications précédentes, **caractérisé en ce que** la cartouche (100) ou l'appareil capteur (113) est conçu pour réaliser un test de séquence d'acide nucléique et/ou un test de protéine.

11. Procédé pour tester un échantillon (P), en particulier un échantillon biologique,
dans lequel un dispositif d'analyse mobile (200) connecte ou reçoit une cartouche (100) avec l'échantillon (P),
dans lequel le dispositif d'analyse (200) commande le test de l'échantillon (P) dans la cartouche (100) et actionne des vannes (115) de la cartouche (100) pour commander l'écoulement à travers les canaux (114) et/ou les cavités (104-111) de la cartouche (100),
dans lequel un appareil capteur (113) de la cartouche (100) détecte ou identifie un ou plusieurs analytes (A) de l'échantillon (P) en liant spécifiquement le ou les analytes (A) à une ou plusieurs molécules de capture (M) et/ou par mesure électrochimique ou redoxcyclage,
dans lequel le dispositif d'analyse (200) détecte ou identifie d'autres analytes (A), composés et/ou caractéristiques matérielles de l'échantillon (P) par mesure optique, de préférence sans liaison spécifique, au moyen d'un spectromètre du dispositif d'analyse (200) fonctionnant indépendamment de l'appareil capteur (113).
